# EUROPÄISCHE PATENTSCHRIFT

(11) **EP 2 405 845 B1**
(45) Veröffentlichungstag und Bekanntmachung des Hinweises auf die Patenterteilung: **25.06.2014**
(21) Anmeldenummer: 10719725.3
(22) Anmeldetag: 09.03.2010
(51) Int. Cl.: A61B 18/12, A61B 18/16, A61B 18/14

(54) **VERFAHREN UND HF-CHIRURGISCHE ANORDNUNG**
METHOD AND HF SURGICAL ARRANGEMENT
PROCÉDÉ ET SYSTÈME CHIRURGICAL HF

(30) Priorität: 10.03.2009 DE 102009012431
(43) Veröffentlichungstag der Anmeldung: 18.01.2012
(73) Patentinhaber: Karl Storz GmbH & Co. KG, 78532 Tuttlingen (DE)
(72) Erfinder: Farin, Günter, 72070 Tübingen (DE)
(74) Vertreter: Lohr, Georg
(86) Internationale Anmeldenummer: PCT/DE2010/075024
(87) Internationale Veröffentlichungsnummer: WO 2010/102620

(56) Entgegenhaltungen:
- DE-A1- 2 514 501
- US-A- 4 244 371
- US-A- 6 113 596
- US-A1- 2008 287 948

## Beschreibung

### Technisches Gebiet

Die Erfindung betrifft ein Verfahren und eine hierfür geeignete HF-chirurgische Anordnung, umfassend bipolare HF-chirurgische Instrumente, insbesondere bipolare Schlingen, mit welchen pathologische Gewebe in Hohlorganen oder Körperhöhlen unter endoskopischer Kontrolle umschlungen und HF-chirurgisch entfernt werden können.

### Stand der Technik

Mit pathologischen Geweben in Hohlorganen sind hier insbesondere Tumore der Mukosa (Schleimhaut) des Gastrointestinaltrakts unter Berücksichtigung einer möglichen Invasion in die jeweils darunter befindliche Submukosa gemeint. Bekannte Verfahren, mit welchen pathologische Mukosa unter endoskopischer Kontrolle mittels hierfür geeigneter Instrumente umschlungen und HF-chirurgisch entfernt bzw. ektomiert oder reseziert werden können, sind die Polypektomie sowie die Mukosektomie oder Mukosaresektion. Die Polypektomie ist anwendbar, wenn das pathologische Gewebe aus dem normalen Mukosaniveau herausragt (allgemein Polyp genannt) und mit einem hierfür verfügbaren Instrument umschlungen und so HF-chirurgisch entfernt werden kann. Ragt ein pathologisches Mukosaareal nicht ausreichend weit aus dem angrenzenden Mukosaniveau heraus um es mit einem verfügbaren Instrument zu umschlingen, dann kann es beispielsweise durch Injektion physiologischer Kochsalzlösung in die darunter befindliche Submukosa so weit aus dem angrenzenden Mukosaniveau herausgehoben werden (allgemein Pseudopolyp genannt), dass es wie ein Polyp umschlungen und entfernt werden kann. Dieses Verfahren wird Mukosektomie oder Mukosaresektion genannt. Gemeinsamer Zweck dieser Verfahren ist die komplette Entfernung pathologischer Mukosa und der darunter befindlichen Submukosa. Dieser Zweck ist erreicht, wenn die pathohistologische Untersuchung des entfernten Gewebes die Erfüllung der Kriterien einer R0 Resektion bestätigt, das heißt, dass die Ektomie bzw. Resektion im gesunden Gewebe außerhalb des pathologischen Gewebes erfolgte, so dass man sicher sein kann, dass das gesamte pathologische Gewebe entfernt wurde.

Hierfür verfügbare HF-chirurgische Instrumente, insbesondere für die endoskopische Polypektomie im Gastrointestinaltrakt, sind die sogenannten Polypektomieschlingen. Mit "Polypektomieschlinge" werden allgemein sowohl die schlingenförmigen Elektroden als auch (pars pro toto) die kompletten HF-chirurgischen Instrumente inklusive der schlingenförmigen Elektroden bezeichnet. Da diese Instrumente nicht nur zur Ektomie von Polypen sondern auch zur Ektomie anderer pathologischer Gewebe, beispielsweise zur Ektomie einer Papilla Vateri (Papillektomie), zur Ektomie oder Resektion pathologischer Mukosa-Areale (Mukosektomie bzw. Mukosaresektion) etc. geeignet sind, werden im Folgenden die schlingenförmigen Elektroden an sich und wo allgemeingültig kurz "Schlinge", die gesamten Instrumente inklusive Schlinge, wo allgemeingültig, kurz "Instrument", die Polypen, Papillen, pathologischen Mukosaareale etc., wo allgemeingültig, kurz "Zielgewebe", und das Ektomieren oder Resezieren etc., wo allgemeingültig, kurz "Entfernen" (Entfernen beinhaltet Schneiden und Koagulieren bzw. thermische Hämostase) genannt.

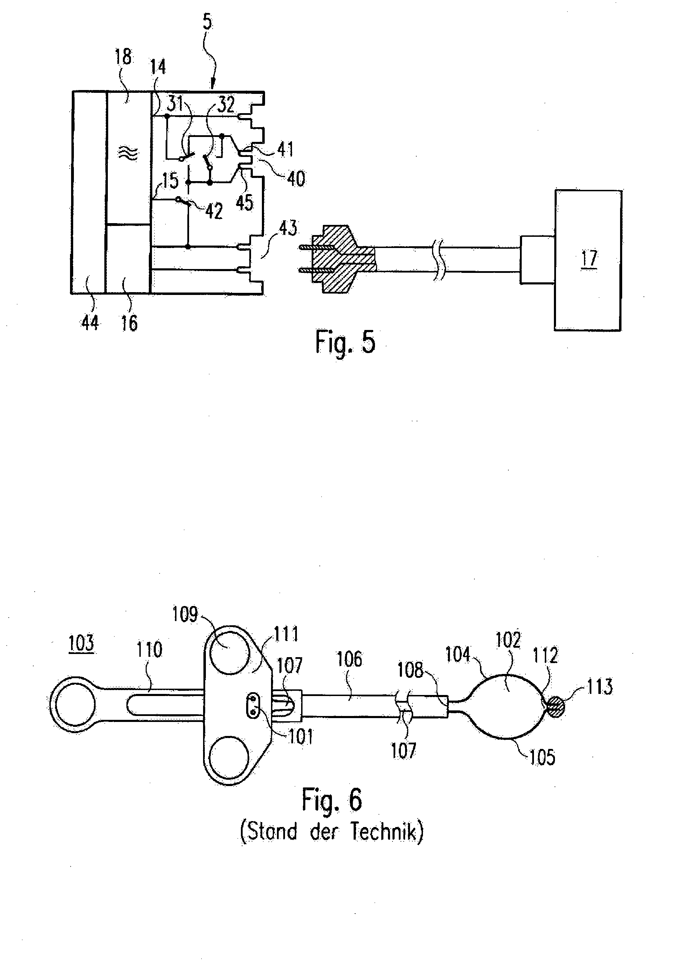

Bekannte Instrumente umfassen, wie in Figur 6 schematisch dargestellt, im Wesentlichen eine Schlinge 102, einen flexiblen Katheter 106, mindestens einen flexiblen aber ausreichend steifen Manipulationsdraht 107, der innerhalb des Katheters in axialer Richtung zum Herausschieben und Hineinziehen der Schlinge aus dem bzw. in das distale Ende 108 des Katheters 106 sowie zum Leiten des zum HF-chirurgischen Entfernen von Zielgeweben erforderlichen HF-Stroms verwendet wird, sowie einen Griff 109 am proximalen Ende des Katheters, der aus einer Gleitschiene 110 und einem Gleiter 111 zum manuellen Herausschieben bzw. Hineinziehen der Schlingen aus dem bzw. in das distale Ende des Katheters besteht. Am Gleiter ist mindestens ein elektrischer Kontakt zum Anschluss eines HF-chirurgischen Generators (HF-Generator) angeordnet. Bipolare Schlingen sind beispielsweise an ihrem distalen Ende 112 in zwei elektrisch voneinander getrennten Schlingenabschnitte getrennt und mechanisch mit einem elektrisch isolierenden Verbindungselement 113 miteinander verbunden Bezüglich der Applikation des HF-Stroms unterscheidet man zwischen sogenannten monopolaren Verfahren und hierfür geeignete Schlingen z.B. DE 2132808, und sogenannten bipolaren Verfahren und hierfür geeignete Schlingen z.B. DE 102007008272 oder DE 3220940.

Monopolare Schlingen sind dadurch gekennzeichnet, dass ihre HF-chirurgisch effektiven Abschnitte nur an einen Pol eines HF-Generators anschließbar sind, während der andere Pol des HF-Generators über eine Neutralelektrode mit dem Patienten verbunden werden muss. Der HF-Strom fließt zwischen Schlinge und Neutralelektrode durch den Patienten, also nicht nur durch das Zielgewebe sondern auch durch anderes bzw. zum Zielgewebe kollaterales Gewebe. Monopolare Schlingen gibt es heute zwar in verschiedenen Formen, wobei diese jedoch keinen beabsichtigten Einfluss auf die HF-chirurgischen Effekte beim Entfernen von Zielgeweben haben, sondern der mechanischen Manipulation bzw. Applikation entsprechend der verschiedenen Lokalisationen, Größen und/oder Formen der Zielgewebe dienen sollen.

Seit inzwischen mehr als 30 Jahren wird darauf hingewiesen, dass bei Anwendung monopolarer Schlingen thermische Schädigungen kollateraler Gewebe durch den zwischen Schlinge und der bei monopolaren Verfahren erforderlichen Neutralelektrode unkontrolliert fließenden HF-Strom verursacht werden können. Genau so lange werden Lösungen zur Vermeidung dieses Problems gesucht und immer wieder werden diesbezüglich bipolare Schlingen als Lösung vorgeschlagen.

Bipolare Schlingen sind dadurch gekennzeichnet, dass die Schlinge, wie beispielsweise die gemäß DE-G 7418576, in zwei gleich lange und spiegelsymmetrisch geformte Schlingenabschnitte (allgemein symmetrische bipolare Schlinge genannt) oder, wie beispielsweise die gemäß DE 3220940, in zwei verschieden lange und in der Regel auch asymmetrisch geformte Schlingenabschnitte geteilt (allgemein asymmetrische bipolare oder quasibipolare Schlingen genannt) und diese Schlingenabschnitte an ihren distalen Enden mit einem die Schlingenabschnitte gegeneinander elektrisch isolierenden Element mechanisch miteinander verbunden sind. Einer der beiden Schlingenabschnitte wird an einem Pol und der andere Schlingenabschnitt an dem anderen Pol eines HF-Generators angeschlossen. In diesem bipolaren Betriebsmodus fließt der HF-Strom vorwiegend zwischen den beiden Schlingenabschnitten durch das zwischen diesen Schlingenabschnitten befindliche Gewebe. Bei symmetrischen bipolaren Schlingen sollen beide Schlingenabschnitte als aktive Elektroden wirken. Bei asymmetrischen bipolaren Schlingen soll der kürzere Schlingenabschnitt als Aktivelektrode und der länger Schlingenabschnitt als Neutralelektrode wirken. Zum Betreiben einer symmetrischen oder asymmetrischen bipolaren Schlinge ist eine separate Neutralelektrode folglich nicht erforderlich. Dementsprechend werden bipolare Schlingen so an die beiden Pole eines HF-Generators angeschlossen, dass die bei HF-Generatoren für monopolaren Betrieb vorgeschriebene automatische Überwachung des Anschlusses einer Neutralelektrode am HF-Generator sowie deren Applikation am Patienten nicht erforderlich oder außer Funktion ist.

Bipolare Schlingen bzw. der bipolare Betrieb sog. bipolarer Schlingen haben sich in der klinischen Anwendung jedoch nicht bewährt. Ein Grund hierfür sind die elektrischen Isolationstrecken am proximalen sowie am distalen Ende zwischen den beiden Schlingenabschnitten. Zum HF-chirurgischen Schneiden muss die Amplitude der HF-Spannung zwischen einer zum Schneiden verwendeten aktiven Elektrode und dem zu schneidenden Gewebe mindestens 200 Volt erreichen. Da bei bipolaren Instrumenten an jeder der beiden aktiven Elektroden gleichzeitig mindestens 200 Volt mit entgegengesetzter Polarität bzw. Phasenlage erreicht werden müssen, müssen die elektrischen Isolationsstrecken zwischen den beiden Aktiven Elektroden insbesondere am proximalen sowie am distalen Ende der Schlinge, wo die Abstände zwischen den beiden Elektroden sehr kurz sind, einer Spannungsamplitude bzw. Amplitudendifferenz von mindestens 400 V standhalten. Entstehen an diesen Stellen elektrische Lichtbogen zwischen den beiden Elektroden, dann können diese Elektroden infolge der Hohen Temperatur dieser elektrischen Lichtbogen schmelzen. Dieses Problem soll entsprechend DE 2514501 durch partielle elektrische Isolation der beiden Schlingenabschnitte gelöst werden, was jedoch am distalen Ende der dort gezeigten bipolaren Schlinge nicht realisert bzw. nicht realisierbar ist.

Seit Einführung der endoskopischen Polypektomie und Mukosektomie möchten Endoskopiker immer größere Polypen bzw. pathologische Mukosa/Submukosa-Areale, insbesondere im Gastrointestinaltrakt, in diagnostischer und/oder therapeutischer Absicht entfernen, und dies mit Rücksicht auf die pathohistologische Untersuchung möglichst in toto und zur Vermeidung von Rezidiven möglichst radikal. Radikal meint hier inklusive der Submukosa des betreffenden Mukosa-Areals bis möglichst nahe der Muskularis propria. Mit der Größe der in toto und radikal ektomierten Polypen bzw. resezierten Mukosa/Submukosa-Areale nehmen allerdings bei Anwendung bisher verfügbarer Polypektomie- bzw. Mukosektomieschlingen auch die hieraus resultierenden Probleme und Komplikationen, insbesondere Blutungen und Perforationen zu. Außerdem können mit diesen Verfahren und den hierfür bisher verfügbaren Polypektomie- bzw. Mukosektomieschlingen sowie den hierfür verfügbaren HF-Generatoren nur Polypen bzw. Mukosa/Submukosa-Areale bis ca. 2 cm Durchmesser in toto entfernt werden. Größere Polypen bzw. Mukosa/Submukosa-Areale können mit diesen Verfahren nur in mehreren kleineren Portionen, der sog. Piecemeal Technik, und oft nicht radikal beziehungsweise vollständig entfernt werden, was die pathohistologische Untersuchung des so entfernten Gewebes und die Zuordnung eines positiven pathologischen Befundes zur jeweiligen Resektionsstelle erschwert oder gar unmöglich macht. Außerdem können nicht vollständig entfernte Tumoren weiter wachsen.

Obwohl die Polypektomie und auch die Mukosektomie heute als klinisch etablierte Verfahren zur Prophylaxe maligner und insbesondere metastasierender Tumoren bewertet werden, sind diese Verfahren noch außer den bereits oben genannten Komplikationen mit Problemen belastet, die auch mit der Größe des Zielgewebes korrelieren.

Eines dieser Probleme, speziell bei großen Polypen und/oder sessilen (flach wachsende) Polypen sowie Mukosa-/Submukosaarealen, die durch submuköse Unterspritzung groß bzw. noch größer werden als sie es bereits sind, sind die für deren in toto Entfernung erforderliche elektrische Leistung bzw. HF-Spannung und HF-Strom. Da der HF-chirurgische Schneideffekt dann und nur dann entstehen kann, wenn zwischen der zum Schneiden verwendeten Schlinge und dem zu schneidenden Gewebe eine Dampfschicht vorhanden ist, so dass bei ausreichend hoher HF-Spannung elektrische Lichtbogen entstehen, die das nahe der Schlinge befindliche Gewebe wegbrennen (Pyrolyse-Effekt), muss das schlingennahe Gewebe auf die Siedetemperatur von Wasser erhitzt werden. Erfolgt die Erhitzung dieses Gewebes zu langsam und entsteht der Schneideffekt folglich zeitlich verzögert (Anschnittverzögerung), so kann die Wärme während der Anschnittverzögerung aus dem schlingennahen Gewebe in benachbarte Gewebe diffundieren und diese thermisch schädigen. Eine thermische Schädigung der Muskularis propria oder gar der Serosa eines Organs des Gastrointestinaltrakts hat in der Regel eine Perforation der Organwand zu Folge.

Für einen ausreichend verzögerungsfreien Anschnitt bei der Polypektomie bzw. Mukosaresektion ist ein HF-Strom von mindestens ca. 0,5 Ampere pro cm Schlingenlänge erforderlich. Da HF-Generatoren von bekannten HF-Chirurgiegeräten maximal 1,5 bis 3 Ampere generieren, können nur Polypen mit einem Durchmesser im Applikationsbereich der Schlinge von ca. 1 bis 2 cm - was einer Schlingenlänge von ca. 3 bis 6 cm entspricht - mit ausreichend geringer Anschnittverzögerung in toto entfernt werden. Bei größeren Polypen bleibt der Schneideffekt gar völlig aus.

Zur Reduzierung des zum Schneiden erforderlichen HF-Stroms und damit auch zur Vermeidung einer Anschnittverzögerung wird in der DE 100 28 413 A1 vorgeschlagen, die wirksame Elektrodenfläche einer monopolaren Schlingenelektrode bekannter Art durch eine Isolierummantelung der beiden Schlingenabschnitte auf einen Teilbereich in der Nähe der Elektrodenspitze zu begrenzen. Die Lehre besteht entsprechend DE 100 28 413 A1 im Wesentlichen darin, die wirksame Elektrodenfläche der Schlingenelektrode durch eine konstruktive Maßnahme nur auf denjenigen Flächenbereich der Schlingenelektrode zu konzentrieren, an welchem der durch den Elektrochirurgiestromfluß erzeugte thermische Schneidevorgang der Schlingenelektrode idealer Weise stattfinden soll. Diese konstruktive Begrenzung der wirksamen Elektrodenfläche mittels eines zumindest teilweisen Isolierens der beiden Schlingenabschnitte soll die Handhabe des elektrochirurgischen Instruments angeblich wesentlich vereinfachen, da die Gefahr von Fehlschnitten oder ungewollten Kontakten mit gesundem Nachbargewebe auf ein Minimum reduziert ist. Letzteres gilt jedoch nur, wenn die Teilbereiche in der Nähe der Elektrodenspitze partiell an ihrem Umfang und/oder an ihrer Spitze isoliert sind, wie es beispielsweise in dieser DE 100 28 413 A1 beschrieben ist, was aber während der Schnittführung störend oder gar hinderlich sein kann. Ein wesentlicher Nachteil der partielle Isolation des Umfangs von Teilbereichen der Schlinge ist jedoch die geringe Effektivität der Koagulation bzw. der thermischen Blutstillung.

In der DE 25 14 501 ist ein bipolares Koagulationsinstrument für Endoskope zum Abtragen von Polypen z.B. im Magen beschrieben, dessen Elektroden ein Hochfrequenzstrom zur Blutstillung zugeleitet wird und das dadurch gekennzeichnet ist, dass die beiden Elektroden eine Schlinge bilden, indem sie an ihren Enden mittels eines Isolierstückes miteinander verbunden sind. Eine Ausgestaltung dieses Coagulationsinstrument ist dadurch gekennzeichnet, dass die Elektroden auf ihrer ganzen Länge mit Ausnahme eines Bereichs in der Nähe des Isolierstückes mit einer Isolierschicht versehen sind. Bei Anwendung eines derartigen bipolaren Coagulationsinstruments sollte es zwar keine Anschnittverzögerung, jedoch Isolationsprobleme im Bereich des Isolierstücks geben, wenn mit diesem Coagulationsinstrument Polypen HF-chirurgisch abgeschnitten werden sollen, wozu hierbei HF-Spannungen mit Amplituden von mindestens 400 V erforderlich sind. Zum Coagulieren bzw. Blutstillen ist die partielle Isolation der Elektroden am Umfang der Enden der Elektroden eher nachteilig weil hierdurch die effektiven Kontaktflächen sehr schmal sind.

In der US 5,078,716 wird eine monopolare Polypektomieschlinge beschrieben, deren beide Schlingenabschnitte proximal bis auf relativ kurze Strecken an deren distalen Enden elektrisch isoliert sind, so dass nur eine relativ kurze Strecke am distalen Ende der Schlinge unisoliert und dadurch HF-chirurgisch effektiv ist. Derartige Schlingen benötigen zwar sowohl während der Anschnittphase als auch während der Schnittphase weniger HF-Strom als gleichgroße Schlingen ohne Isolation, haben jedoch den Nachteil, dass HF-chirurgisch effektive Teil der Schlinge aus der Perspektive eines Endoskops immer hinter dem Polypen, also außer Sichtkontrolle ist und das Risiko besteht, dass insbesondere die distale Spitze der Schlinge unkontrolliert insbesondere dünnwandige Organe perforieren kann. Letzteres zu vermeiden ist unter anderem Gegenstand der DE 100 28 413 A1.

Sowohl bei elektrochirurgischen Instrumenten nach US 5,078,716, DE 100 28 413 A1 als auch bei Coagulationsistrumenten entsprechend DE 25 14 501 können die HF-chirurgisch wirksamen Elektrodenflächen nur am distalen Ende der Schlinge angeordnet werden, mit dem Nachteil, dass diese aus der Blickrichtung eines Endoskops immer hinter dem zu entfernenden Gewebe und folglich ohne Sichtkontrolle aktiv werden.

Ein weiteres Problem besteht darin, dass große Zielgewebe mit bisher verfügbaren Schlingen und HF-Generatoren nicht in toto, sondern nur in mehreren kleineren Portionen (piecemeal technique) von einer Organwand entfernt werden können. Zur in toto Entfernung größerer Mukosa/Submukosa-Areale werden zurzeit verschiedene endoskopische Submukosa Dissektions (ESD) Verfahren entwickelt und einige auch bereits klinisch angewendet. Gemeinsames Merkmal dieser ESD-Verfahren ist das HF-chirurgische Abpräparieren des betreffenden Mukosa-/Submukosa-Areals nahe der Muskularis propria, beispielsweise mit einer Nadelelektrode. Diese Verfahren setzen eine hohe manuelle Geschicklichkeit, Erfahrung, ständiges Training, Risikobereitschaft und viel Zeit voraus. Bisher gibt es nur wenige Experten, die diese Verfahren praktizieren.

Die US 2008/287948 A1 offenbart ein hochfrequenzchirurgisches System, bei dem ein Hochfrequenzchirurgiegenerator eine bipolare Elektrode, wahlweise für monopolaren oder bipolaren Betrieb, speist.

In der US 4,244,371 ist ein Hochfrequenzchirurgiegenerator für monopolare und bipolare Betriebsarten offenbart.

In der US 6,113,596 ist ein hochfrequenzchirurgisches System offenbart, mit dem monopolare und bipolare Instrumente betrieben werden können.

Zusammenfassend kann hier zum Stand der Technik bemerkt werden, dass für die endoskopische Entfernung von Zielgeweben inzwischen zwar viele verschieden konstruierte Schlingen verfügbar sind oder zumindest vorgeschlagen wurden (siehe oben), bisher jedoch keine hiervon geeignet ist, größere Zielgewebe, insbesondere größere als 2 cm im Durchmesser, in toto ausreichend sicher zu entfernen.

### Darstellung der Erfindung

Es ist Aufgabe der Erfindung, HF-chirurgische Verfahren und hierfür geeignete Einrichtungen für die endoskopisch kontrollierte Entfernung pathologischer Gewebe, insbesondere der Mukosa und Submukosa des Gastrointestinaltrakts, aufzuzeigen, bei deren Anwendung die oben aufgeführten Probleme weniger oder gar nicht vorhanden sind und mit denen insbesondere auch größere Gewebeareale als ca. 2 cm im Durchmesser in toto und möglichst radikal von Organwänden entfernt werden können, die bisher der Piecemeal Technik, der endoskopischen Submukosa Dissektion (ESD) oder der offenen Chirurgie vorbehalten sind.

Diese Aufgabe wird verfahrensmäßig gelöst, indem sogenannte bipolare Schlingen, also Schlingen mit zwei elektrisch gegeneinander isolierten Schlingenabschnitten, angewendet werden, diese jedoch nicht nur bipolar, sondern alternativ oder alternierend auch monopolar betrieben werden indem nur einer oder gleichzeitig beide der zwei Schlingenabschnitte alternativ oder alternierend an einen Pol eines HF-Generators als Aktivelektrode angeschlossen wird bzw. werden, während der andere Pol des HF-Generators mit einer am Patienten applizierten Neutralelektrode verbunden ist.

Insbesondere wird diese Aufgabe im Hinblick auf das Verfahren durch den Gegenstand des Anspruchs 1 und im Hinblick auf die Einrichtung durch den Gegenstand des Anspruchs 8 gelöst.

Die Erfindung beruht auf dem Gedanken, ein Verfahren zum Betreiben einer HF-chirurgischen Anordnung zum HF-chirurgischen Entfernen pathologischer Gewebe in Hohlorganen eines Patienten, z.B. von Polypen im Gastrointestinaltrakt, anzugeben, wobei die HF-chirurgische Anordnung ein HF-Chirurgiegerät mit einem HF-Generator zur Erzeugung des hierfür erforderlichen HF-Stroms und eine bipolare Schlinge umfasst, die zwei gegeneinander isolierte Schlingenabschnitte aufweist, die mit dem Gewebe elektrisch in Kontakt bringbar sind und wobei der HF-Generator einen ersten und einen gegenpoligen zweiten Ausgang aufweist. Dabei wird erfindungsgemäß in einem bipolaren Betrieb jeweils einer der Ausgänge mit jeweils einem Schlingenabschnitt verbunden und in einem monopolaren Betrieb der erste Ausgang mit einer am Patienten anbringbaren Neutralelektrode und der zweite Ausgang mit mindestens einem Schlingenabschnitt verbunden, wobei weiterhin mindestens zeitweise ein monopolarer Betrieb eingestellt wird, bei welchem nur einer der beiden Schlingenabschnitte mit dem zweiten Ausgang verbunden ist.

Der monopolare Betrieb einer bipolaren Schlinge mit nur einem Schlingenabschnitt als Aktivelektrode ermöglicht die Entfernung auch großer Polypen bzw. Mukosa-Submukosa-Areale in toto, die mit monopolaren Schlingen nicht in toto entfernt werden können, und dies außerdem ohne Anschnittprobleme und ohne Überlastung der elektrischen Isolationsstrecken zwischen den beiden Schlingenabschnitten. Bei sehr großen Polypen bzw. Mukosa-Submukosa-Arealen können vorzugsweise asymmetrische bipolare Schlingen angewendet werden, wobei vorzugsweise der jeweils kürzere Schlingenabschnitt als Aktivelektrode verwendet wird.

Der monopolare Betrieb einer bipolaren Schlinge mit gleichzeitig beiden Schlingenabschnitten als Aktivelektrode ermöglichst die Entfernung von Polypen bzw. Mukosa-Submukosa-Areale, die auch mit monopolaren Schlingen in toto und ohne Anschnittprobleme entfernt werden können. Die Anwendung bipolarer statt monopolarer Schlingen kann in diesen Fällen trotzdem vorteilhaft sein, wenn hierdurch für ein Krankenhaus die Beschaffungs- und Bevorratungskosten durch Beschränkung auf möglichst wenige Schlingen Typen reduziert werden können.

Darüber hinaus bietet der alternativ monopolare Betrieb bipolarer Schlingen weitere Vorteile im Vergleich sowohl zu bekannten teilisolierten als auch zu konventionellen nicht teilisolierten monopolaren Schlingen, so beispielsweise die bessere Kontrollierbarkeit der Schnittrichtung, die bessere endoskopische Sicht auf die HF-chirurgisch aktiven Schlingenabschnitte und insbesondere die Möglichkeit, den HF-chirurgischen Schnitt monopolar mit nur einem oder beiden der zwei Schlingenabschnitte und die thermische Blutstillung, allgemein Koagulation genannt, bipolar zwischen beiden Schlingenabschnitten durchzuführen, wobei das bipolare Koagulieren mit der sogenannten Softkoagulation durchgeführt werden kann, die dadurch gekennzeichnet ist, dass die Amplitude der hierfür erforderlichen HF-Spannung kleiner als 200 Volt ist, so dass die kritischen Isolationsstrecken bipolarer Schlingen nicht überlastet werden.

Dieses Verfahren ist jedoch mit bisher verfügbaren Einrichtungen und/oder HF-Chirurgiegeräten nicht anwendbar. Bisher verfügbare HF-Chirurgiegeräte haben zwar mindestens eine Anschlussbuchse zum monopolaren Betreiben monopolarer Instrumente inklusive monopolarer Schlingen und/oder mindestens eine Anschlussbuchse zum bipolaren Betreiben bipolare Instrumente inklusive bipolarer Schlingen, jedoch keine Anschlussbuchse zum monopolaren Betreiben bipolarer Schlingen.

Der Erfindung liegt daher auch der Gedanke zugrunde, eine Einrichtung zum Betreiben einer HF-chirurgischen Anordnung zum HF-chirurgischen Entfernen pathologischer Gewebe in Hohlorganen eines Patienten, z.B. von Polypen im Gastrointestinaltrakt, anzugeben, wobei die HF-chirurgische Anordnung ein HF-Chirurgiegerät mit einem HF-Generator zur Erzeugung des hierfür erforderlichen HF-Stroms und eine bipolare Schlinge umfasst, die zwei gegeneinander isolierte Schlingenabschnitte aufweist, die mit dem Gewebe elektrisch in Kontakt bringbar sind und wobei der HF-Generator einen ersten und einen gegenpoligen zweiten Ausgang aufweist. Die Einrichtung umfasst dabei Adapter, Schalter und/oder Wechselschalter oder dergleichen Umschalteinrichtung, die in einem bipolaren Betrieb jeweils einen der Ausgänge mit jeweils einem Schlingenabschnitt verbindet und die in einem monopolaren Betrieb den ersten Ausgang mit einer am Patienten anbringbaren Neutralelektrode und den zweiten Ausgang mit mindestens einem Schlingenabschnitt verbindet und die weiterhin derart ausgebildet ist, dass mindestens zeitweise ein monopolarer Betrieb einstellbar ist, bei welchem nur einer der beiden Schlingenabschnitte mit dem zweiten Ausgang verbunden ist.

Die erfindungsgemäße Einrichtung zum monopolaren oder sowohl zum monopolaren als auch zum bipolaren Betreiben sogenannter bipolarer Schlingen, also Schlingen mit zwei elektrisch gegeneinander isolierten Schlingenabschnitten, umfasst vorzugsweise elektrische Verbindungselemente, insbesondere steckbare und/oder schaltbare Kontaktelemente, womit zum monopolaren Betreiben bipolarer Schlinge nur einer oder alternativ oder alternierend der eine oder der andere oder gleichzeitig beide Schlingenabschnitte gemeinsam mit einem der beiden Pole eines HF-Generator eines HF-Chirurgiegeräts verbunden werden können und der anderer Pol des HF-Generators des HF-Chirurgiegeräts, wie bei monopolaren Betriebsmodi üblich, durch Verbindungselemente mit einer Neutralelektrode verbunden werden kann bzw. wird, oder womit alternativ zu oder alternierend mit mindestens einem der oben aufgeführten monopolaren BetriebsModi zum bipolaren Betreiben bipolarer Schlingen der eine Pol des HF-Generators mit dem einen Schlingenabschnitt und der andere Pol des HF-Generators statt mit einer Neutralelektrode mit dem anderen Schlingenabschnitt verbunden werden kann bzw. wird.

### Beschreibung der Zeichnungen

Im Folgenden werden anhand von Prinzipschaltbildern verschiedene Ausführungsbeispiele erfindungsgemäßer Einrichtungen beschrieben. Identische bzw. gleich wirkende Elemente sind mit identischen Kennziffern gekennzeichnet. Es zeigen
- Figur 1: einen Adapter zum monopolaren Betreiben bipolarer Schlingen,
- Figur 2: ein Adapterkabel zum monopolaren Betreiben bipolarer Schlingen,
- Figur 3a: eine Umschalteinrichtung zum monopolaren Betreiben bipolarer Schlingen,
- Figur 3b: eine HF-Chirurgiegerät mit einer Einrichtung zum monopolaren Betreiben bipolarer Schlingen,
- Figur 4: ein HF-Chirurgiegerät mit einer Einrichtung zum monopolaren oder bipolaren Betreiben bipolarer Schlingen und
- Figur 5: ein HF-Chirurgiegerät mit einer Einrichtung zum monopolaren oder bipolaren Betreiben bipolarer Schlingen.

Ein erstes Ausführungsbeispiel einer erfindungsgemäßen Einrichtung zum monopolaren Betreiben sog. bipolarer Schlingen 103, also Schlingen mit zwei elektrisch gegeneinander isolierten Schlingenabschnitten 104, 105, umfasst elektrische Verbindungselemente, womit zum monopolaren Betreiben dieser Schlingen 103 jeweils nur einer der zwei Schlingenabschnitte 104, 105 mit einem der beiden Ausgänge 14, 15 eines HF-Generators 18 verbunden werden kann, und der andere Ausgang 14, 15 des HF-Generators 18 bei Anwendung dieser Einrichtung, wie bei monopolaren Betriebsmodi üblich, in bekannter Weise mit einer Neutralelektrode 17 verbunden werden oder sein muss.

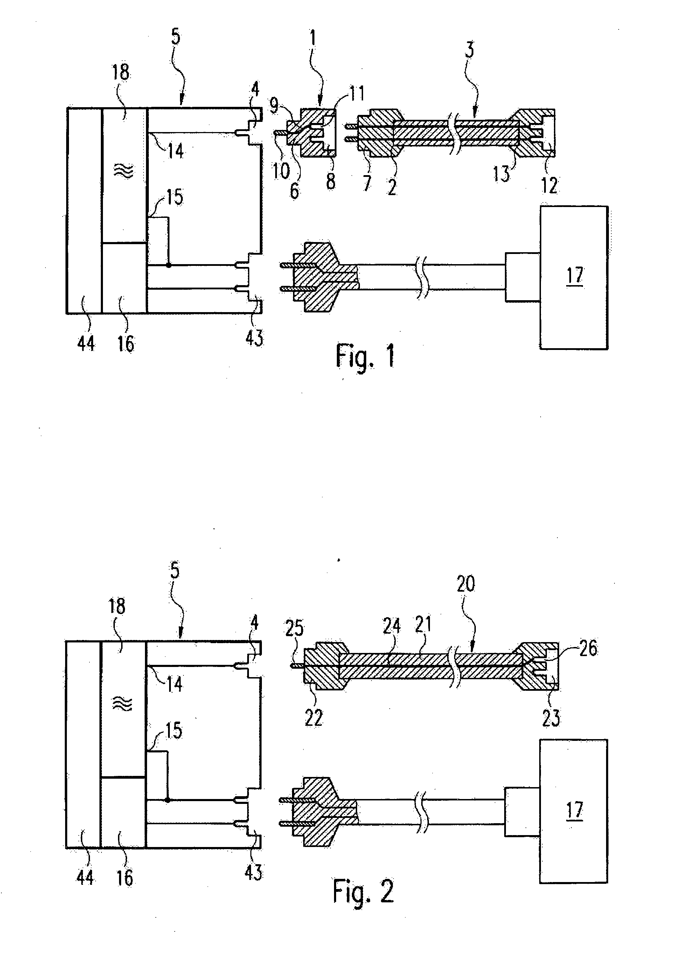

Eine Einrichtung entsprechend diesem Ausführungsbeispiel kann, wie in Fig. 1 schematisch dargestellt, einen Adapter 1 umfassen, der beispielsweise zwischen dem proximalen Ende 2 eines bipolaren Anschlusskabels 3 für bipolare Schlingen 103 bzw. Instrumente und einer monopolaren Anschlussbuchse 4 eines HF-Chirurgiegeräts 5 eingefügt werden kann. Der Adapter 1 umfasst zwei Anschlusselemente, und zwar einen zur monopolaren Anschlussbuchse 4 des HF-Chirurgiegeräts 5 kompatiblen monopolaren Anschlussstecker 6 und eine zum bipolaren Anschlussstecker 7 am proximalen Ende 2 des zweiadrigen bzw. bipolaren Anschlusskabels 3 kompatible bipolare Anschlussbuchse 8. Eine elektrisch leitfähige Brücke 9 verbindet ein erstes Kontaktelement 10 des monopolaren Anschlusssteckers 6 mit einem zweiten Kontaktelement 11 der bipolaren Anschlussbuchse 8.

Die Verbindungselemente, insbesondere der bipolare Anschlussstecker 7 und die bipolare Anschlussbuchse 8, zwischen einem derartigen Adapter 1 und einem bipolaren Anschlusskabel 3 und/oder einer weiteren bipolaren Anschlussbuchse 12 am distalen Ende 13 des bipolaren Anschlusskabels 3 und dem bipolaren Steckkontakt 101 bipolarer Schlingen 103 bzw. Instrumente (s. Fig. 6) können so gestaltet sein, dass exklusive nur der eine oder alternativ der eine oder der andere der zwei Schlingenabschnitte 104, 105 mit einem der beiden Ausgänge 14, 15 des HF-Generators 18 des HF-Chirurgiegeräts 5 verbunden werden kann, beispielsweise indem die Steckrichtung des bipolaren Anschlusssteckers 7 am proximalen Ende 2 und/oder distalen Ende 13 des bipolaren Anschlusskabels 3 fix oder variabel gestaltet ist. Dieser Aspekt ist insbesondere bei Anwendung asymmetrischer bipolarer Schlingen 103 relevant. Bei Anwendung asymmetrischer bipolarer Schlingen 103 ist es insbesondere zum in toto Entfernen sehr großer Zielgewebe zweckmäßig, den jeweils kürzeren Schlingenabschnitt 104, 105 als Aktivelektrode zu benutzen.

Diese Einrichtung zeichnet sich folglich dadurch aus, dass sie exklusiv nur einen oder alternativ den einen oder den anderen der zwei Schlingenabschnitte 104, 105 einer bipolaren Schlinge 103 mit dem Ausgang 14, 15 eines HF-Generators verbinden kann, an den im monopolaren Betriebsmodus bestimmungsgemäß monopolare Aktivelektroden bzw. Schlingenabschnitte 104, 105 angeschlossen werden. Bei Anwendung asymmetrischer bipolarer Schlingen 103 ist es insbesondere zum in toto Entfernen sehr großer Zielgewebe zweckmäßig, den jeweils kürzeren Schlingenabschnitt 104, 105 als Aktivelektrode zu benutzen. Abweichend vom bipolaren Betrieb einer bipolaren Schlinge 103 muss bei monopolarem Betrieb einer bipolaren Schlinge 103 bei Anwendung dieser Einrichtung eine Neutralelektrode 17 sowohl an den ersten Ausgang 15 des HF-Generators 18 angeschlossen als auch am Patienten appliziert sein und dies von einer hierfür üblichen, in HF-Chirurgiegeräten 5 vorschriftsmäßig enthaltenen und deswegen nicht detaillierter dargestellten und beschriebenen automatischen Sicherheitseinrichtung 16, beispielsweise entsprechend EP 0 390 937, kontrolliert werden.

Ein zweites Ausführungsbeispiel einer erfindungsgemäßen Einrichtung zum monopolaren Betreiben sog. bipolarer Schlingen 103, also Schlingen 103 mit zwei elektrisch gegeneinander isolierten Schlingenabschnitten 104, 105, umfasst elektrische Verbindungs-Elemente, womit zum monopolaren Betreiben der Schlinge 103 nur einer der zwei Schlingenabschnitte 104, 105 mit einem der beiden Ausgänge 14, 15 eines HF-Generators 18 eines HF-Chirurgiegeräts 5 verbunden werden kann, und der andere Ausgang 15, 14 des HF-Generators 18 bei Anwendung dieser Einrichtung, wie bei monopolaren Betriebsmodi üblich, in bekannter Weise mit einer Neutralelektrode 17 verbunden werden oder sein muss.

Eine Einrichtung entsprechend diesem Ausführungsbeispiel kann, wie in Fig. 2 schematisch dargestellt, einen weiteren Adapter bzw. ein Adapterkabel 20 umfassen, das beispielsweise auch als Verlängerungskabel zwischen dem proximalen Ende 2 eines in der Regel zweiadrigen, sog. bipolaren Anschlusskabels 3 für bipolare Schlingen 103 oder dem bipolaren Steckkontakt 101 einer bipolaren Schlinge 103 bzw. eines bipolaren Instruments und einer monopolaren Anschlussbuchse 4 eines HF-Chirurgiegeräts 5 eingefügt werden kann. Das Adapterkabel 20 umfasst einen Kabelabschnitt 21 mit je einem elektrischen Anschlusselement an dessen Enden, und zwar einem zur monopolaren Anschlussbuchse 4 des HF-Chirurgiegeräts 5 kompatiblen Verbindungsstecker 22 und eine zum bipolaren Anschlussstecker 7 am proximalen Ende 2 des bipolaren Anschlusskabel 3 oder zum bipolaren Steckkontakt 101 direkt an der bipolaren Schlinge 103 bzw. am bipolaren Instrument kompatible Verbindungsbuchse 23. Eine elektrisch leitfähige Ader 24 des Adapterkabels 20 verbindet ein erstes Kontaktelement 25 des Verbindungssteckers 22 mit einem Kontaktelement 26 der Verbindungsbuchse 23 dieses Adapterkabels 20.

Ist die Steckrichtung zwischen der Verbindungsbuchse 23 am distalen Ende des Adapterkabels 20 und dem bipolaren Anschlussstecker 7 am proximalen Ende 2 des als Verlängerungskabel verwendbaren bipolaren Anschlusskabels 3 variabel, dann kann alternativ der eine oder der andere Schlingenabschnitt 104, 105 mit dem zweiten Ausgang 14 des HF-Generators 18 verbunden werden, was insbesondere bei Anwendung asymmetrischer bipolarer Schlingen 103 vorteilhaft sein kann.

Diese Einrichtung ist folglich dadurch gekennzeichnet, dass sie exklusiv nur einen oder, wenn die Steckrichtung mindestens einer Steckverbindung, insbesondere zwischen dem bipolaren Anschlussstecker 7 bzw. dem bipolaren Steckkontakt 101 und der Verbindungsbuchse 23, variabel ist, alternativ den einen oder den anderen der zwei Schlingenabschnitte 104, 105 einer bipolaren Schlinge 103 mit dem zweiten Ausgang 14 eines HF-Generators 18 verbinden kann, an den im monopolaren Betriebsmodus monopolare Aktivelektroden angeschlossen werden. Abweichend vom bipolaren Betrieb einer bipolaren Schlinge 103 muss bei monopolarem Betrieb einer bipolaren Schlinge 103, wie bereits oben beim ersten Ausführungsbeispiel bemerkt, auch bei monopolarem Betrieb einer bipolaren Schlinge 103 mit dieser Einrichtung eine Neutralelektrode 17 sowohl an den ersten Ausgang 15 des HF-Generator 18 angeschlossen als auch am Patienten appliziert sein und dies von einer hierfür üblichen und deswegen nicht detaillierter dargestellten und beschriebenen automatischen Sicherheitseinrichtung 16 kontrolliert werden.

Die oben anhand von Fig. 1 und Fig. 2 beschriebenen Ausführungsbeispiele von Einrichtungen zum alternativ monopolaren Betreiben bipolarer Schlingen 103 sind insbesondere für die vielen bereits vorhandenen HF-Chirurgiegeräte 5 zweckmäßig, die noch keine Einrichtung zum monopolaren Betrieb nur eines der zwei Schlingenabschnitte 104, 105 bipolarer Schlingen 103 haben. Selbstverständlich kann eine zum bipolaren Anschlussstecker 7 bzw. Verbindungsstecker 22 des bipolaren Anschlusskabels 3 bzw. des Adapterkabels 20 kompatible bipolare Anschlussbuchse 8 auch integraler Bestandteil eines HF-Chirurgiegeräts 5 sein, wobei jedoch für den monopolaren Betrieb bipolarer Schlingen 103 nur einer der beiden Ausgänge 14, 15 des HF-Generators 18 mit einem oder beiden Kontakten dieser Anschlussbuchse 8 verbunden sein darf und außerdem eine zum monopolaren Betrieb erforderliche Neutralelektrode 17 am Patienten appliziert und am HF-Generator 18 angeschlossen sein muss. HF-Chirurgiegeräte 5, die zum bipolaren Betrieb bipolarer Schlingen 103 bereits mit einer zu bipolaren Anschlusskabeln 3 kompatiblen bipolaren Anschlussbuchse 8 ausgestattet sind, können jedoch nicht allein dadurch zum monopolaren Betrieb bipolarer Schlingen 103 modifiziert werden, indem einer der beiden Ausgänge 14, 15 des HF-Generators 18 von dieser bipolaren Anschlussbuche 8 getrennt wird. Bekannte HF-Chirurgiegeräte 5 mit integrierten bipolaren Anschlussbuchsen 8 für bipolare Schlingen 103 bzw. Instrumente sind nämlich dadurch gekennzeichnet, dass im bipolaren Betriebs-Modus die bei HF-Chirurgiegeräten 5 vorschriftsmäßige automatische Kontrolle des Anschlusses einer Neutralelektrode 17 am HF-Generator 18 und darüber hinaus die automatische Kontrolle der Applikation von Neutralelektroden 17 am Patienten deaktiviert wird.

Ein drittes Ausführungsbeispiel einer erfindungsgemäßen Einrichtung zum monopolaren Betreiben sog. bipolarer Schlingen 103, also Schlingen 103 mit zwei elektrisch gegeneinander isolierten Schlingenabschnitten 104, 105, umfasst elektrische Verbindungselemente, womit zum monopolaren Betreiben dieser Schlingen 103 alternativ oder alternierend der eine oder der andere der zwei Schlingenabschnitte 104,105, oder alternativ oder alternierend der eine oder der andere oder beide Schlingenabschnitte 104, 105 gleichzeitig mit einem der beiden Ausgänge 14, 15 eines HF-Generators 18 verbunden werden kann, und der andere Ausgang 15, 14 des HF-Generators 18 bei Anwendung dieser Einrichtung, wie bei monopolaren Betriebsmodi üblich, in bekannter Weise mit einer Neutralelektrode 17 verbunden werden oder sein muss.

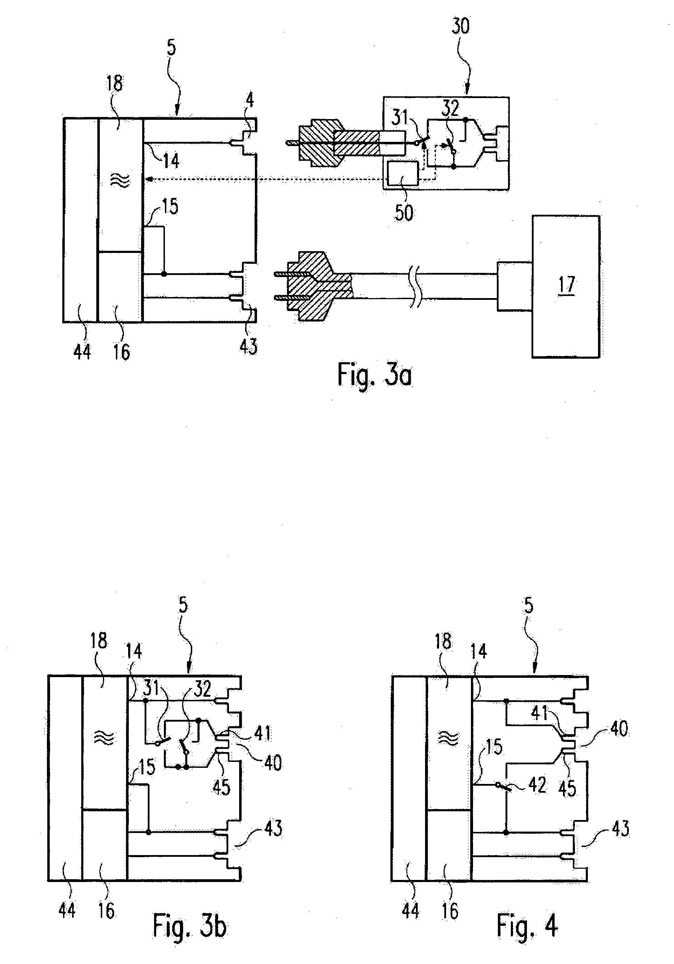

Eine Einrichtung entsprechend diesem Ausführungsbeispiel kann beispielsweise, wie in Fig. 3a schematisch dargestellt, eine Umschalteinrichtung 30 umfassen und bestimmungsgemäß als Switch Box verwendet werden, oder sie kann, wie in Fig. 3b schematisch dargestellt, integraler Bestandteil eines HF-Chirurgiegeräts 5 sein. In Form einer Umschalteinrichtung 30 unterscheidet sich dieses Ausführungsbeispiel im Vergleich zum ersten Ausführungsbeispiel dadurch, dass zusätzlich ein erster Wechselschalter 31 vorhanden ist, durch welchen alternativ oder alternierend der eine oder der andere Schlingenabschnitt 104, 105 einer bipolaren Schlinge 103 mit dem zweiten Ausgang 14 des HF-Generator 18 verbunden werden kann. Die Umschalteinrichtung 30 kann zusätzlich einen Schalter 32 umfassen, durch welchen die beiden Schlingenabschnitte 104, 105 elektrisch miteinander verbunden werden können.

Ein viertes Ausführungsbeispiel einer erfindungsgemäßen Einrichtung, das alternativ oder alternierend sowohl zum monopolaren als auch zum bipolaren Betreiben sog. bipolarer Schlingen 103, also Schlingen 103 mit zwei elektrisch gegeneinander isolierten Schlingenabschnitten 104, 105, geeignet ist, umfasst elektrische Verbindungselemente zum elektrisch leitfähigen Verbinden eines Ausgangs 14, 15 des HF-Generators 18 mit einem der zwei Schlingenabschnitte 104, 105, und einen ersten Wechselschalter 31 zum alternativen oder alternierenden Verbinden des anderen Ausgangs 15, 14 des HF-Generators 18 mit dem anderen der zwei Schlingenabschnitte 105, 105 oder mit einer Neutralelektrode 17.

Eine Switch Box bzw. eine Umschalteinrichtung 30 entsprechend diesem Ausführungsbeispiel kann gemäß Fig. 3a an HF-Chirurgiegeräten 5 ohne Mittel zum monopolaren Betreiben bipolarer Schlingen 103 verwendet werden, ist aber vorteilhafter ein integraler Bestandteil eines HF-Chirurgiegeräts 5 und wird deswegen anhand von Fig. 4 auch nur in dieser Ausführungsform dargestellt und beschrieben. Einem Fachmann auf diesem Gebiet ist es sicher möglich, diese Ausführungsform auch in Form einer Switch Box oder dergleichen zu realisieren.

Als integraler Bestandteil eines HF-Chirurgiegeräts 5 umfasst dieses Ausführungsbeispiel, wie in Fig. 4 schematisch dargestellt, im Wesentlichen eine zum bipolaren Anschlussstecker 7 am proximalen Ende 2 des bipolaren Anschlusskabels 3 kompatible bipolare Zusatzbuchse 40, deren erster Kontakt 41 permanent mit dem zweiten Ausgang 14 des HF-Generators 18 verbunden ist, und einen zweiten Wechselschalter 42, durch den der erste Ausgang 15 des HF-Generators 18 alternativ oder alternierend mit dem anderen der zwei Schlingenabschnitte 104, 105 oder mit einer Neutralanschlussbuchse 43 angeschlossenen Neutralelektrode 17 verbunden werden kann. Auch dieses Ausführungsbeispiel kann nur bestimmungsgemäß angewendet werden, wenn eine Neutralelektrode 17 am HF-Chirurgiegerät 5 angeschlossen und am Patienten appliziert ist. Ist das HF-Chirurgiegerät 5 mit einer Sicherheitseinrichtung 16 zur automatischen Kontrolle des Anschlusses einer Neutralelektrode 17 am HF-Chirurgiegerät 5 und/oder der Applikation der Neutralelektrode 17 am Patienten ausgestattet, dann ist diese Sicherheitseinrichtung 16 ohne oder unzureichend applizierter Neutralelektrode 17 nicht anwendbar.

Ein fünftes Ausführungsbeispiel einer erfindungsgemäßen Einrichtung, das zum monopolaren oder sowohl zum monopolaren als auch zum bipolaren Betreiben sogenannter bipolarer Schlingen 103, also Schlingen 103 mit zwei elektrisch gegeneinander isolierten Schlingenabschnitten 104, 105 geeignet ist, umfasst elektrische Verbindungselemente, womit zum monopolaren Betreiben bipolarer Schlingen 103 einer der beiden Ausgänge 14, 15 eines HF-Generators 18 mit nur einer oder alternativ oder alternierend mit dem einen oder dem anderen oder gleichzeitig mit beiden der zwei Schlingenabschnitte 104, 105 gemeinsam verbunden werden kann bzw. können, und der andere Ausgang 15, 14 des HF-Generators 18 alternativ oder alternierend mit dem anderen der zwei Schlingenabschnitte 104, 105 oder mit einer Neutralelektrode 17 verbunden werden kann.

Dieses in Fig. 5 dargestellte fünfte Ausführungsbeispiel ist im Wesentlichen eine Kombination der bereits oben anhand der Fig. 3b und Fig. 4 dargestellten und beschriebenen Ausführungsbeispiele und enthält keine zusätzlichen Verbindungselemente zu den bereits oben anhand der Fig. 3b und Fig. 4 dargestellten und beschrieben Ausführungsbeispielen. Eine Beschreibung dieses Ausführungsbeispiels anhand von Fig. 5 wäre für einen Fachmann auf diesem Gebiet eine Wiederholung der Beschreibungen des dritten und vierten Ausführungsbeispiels.

Anhand des in Figur 5 dargestellten fünften Ausführungsbeispiels, und dies gilt ergänzend auch für die bereits oben anhand von Fig. 3b und Fig. 4 beschriebenen Ausführungsbeispiele, werden im Folgenden jedoch weitere Ausgestaltungen dieser Einrichtungen, insbesondere bezüglich ihrer Funktion und Gebrauchstauglichkeit beschrieben.

Eine Ausgestaltung der in den Fig. 3b und Fig. 4 dargestellten Ausführungsbeispiele und insbesondere deren in Fig. 5 als fünftes Ausführungsbeispiel dargestellten Kombination besteht darin, dass die schaltbaren Verbindungselemente, also insbesondere Schalter 32 und Wechselschalter 31, 42, elektromechanische Relais oder auch Halbleiterschalter sind, deren Schaltstellungen elektronisch, und dies insbesondere durch speicherbare bzw. gespeicherte Programme und hierfür geeignete und einem Durchschnittsfachmann auf diesem Gebiet hinlänglich bekannte elektronische Steuereinrichtungen 50, beispielweise durch einen elektronischen Rechner bzw. eine CPU 44, alternativ oder alternierend konfiguriert werden können. Diese Ausgestaltung ist für bestimmungsgemäße Anwendungen dieser Einrichtungen, beispielsweise für die endoskopisch kontrollierte Entfernung insbesondere großer und folglich komplikationsträchtiger Polypen bzw. pathologischer Mukosa-Submukosa-Areale von dünnwandigen Hohlorganen, insofern sehr vorteilhaft, als die Entfernung derartiger Zielgewebe mehrere verschiedene Phasen mit spezifischen Anforderungen bezüglich der jeweils erforderlichen thermischen Effekte umfasst, deren zeitlich schnelle Abfolge durch manuelles Modifizieren der Verbindungselemente nicht beherrschbar ist.

Außerdem können insbesondere Einrichtungen entsprechend dem fünften Ausführungsbeispiel bzw. Fig. 5 sehr vorteilhaft mit speziellen Betriebsmodi bzw. Programmen, beispielsweise entsprechend DE 35 30 335 für die Polypektomie, betrieben werden, derart dass die Konfiguration der elektromechanisch schaltbaren Verbindungselemente automatisch beispielsweise mit den alternierenden Schneide- und Koagulations-Phasen synchronisiert wird. So können beispielsweise zyklisch Schnitt-Phasen alternierend und monopolar mit dem einen und/oder mit dem anderen der zwei Schlingenabschnitte 104, 105, und Koagulations-Phasen entweder monopolar mit dem einen und/oder mit dem anderen der zwei Schlingenabschnitte 104, 105, oder bipolar mit Soft-Koagulation zwischen beiden Schlingenabschnitten 104, 105 bipolarer Schlingen 103 angewendet werden.

### Bezugszeichenliste

- 1: Adapter
- 2: Proximales Ende
- 3: Bipolares Anschlusskabel
- 4: Monopolare Anschlussbuchse
- 5: HF-Chirurgiegerät
- 6: Monopolarer Anschlussstecker
- 7: Bipolarer Anschlussstecker
- 8: Bipolare Anschlussbuchse
- 9: Brücke
- 10: Erstes Kontaktelement
- 11: Zweites Kontaktelement
- 12: Weitere bipolare Anschlussbuchse
- 13: Distales Ende
- 14: Zweiter Ausgang
- 15: Erster Ausgang
- 16: Sicherheitseinrichtung
- 17: Neutralelektrode
- 18: HF-Generator
- 20: Adapterkabel
- 21: Kabelabschnitt
- 22: Verbindungsstecker
- 23: Verbindungsbuchse
- 24: Ader
- 25: Erstes Kontaktelement
- 26: Zweites Kontaktelement
- 30: Umschalteinrichtung
- 31: Erster Wechselschalter
- 32: Schalter
- 40: bipolare Zusatzbuchse
- 41: erster Kontakt
- 42: zweiter Wechselschalter
- 43: Neutralanschlussbuchse
- 44: CPU
- 45: zweiter Kontakt
- 50: Steuereinrichtung
- 101: Bipolarer Steckkontakt

## Patentansprüche

1. Verfahren zum Betreiben einer HF-chirurgischen Anordnung zum HF-chirurgischen Entfernen pathologischer Gewebe in Hohlorganen eines Patienten, z.B. von Polypen im Gastrointestinaltrakt, wobei die HF-chirurgische Anordnung ein HF-Chirurgiegerät (5) mit einem HF-Generator (18) zur Erzeugung des hierfür erforderlichen HF-Stroms und eine bipolare Schlinge (103) umfasst, die zwei gegeneinander isolierte Schlingenabschnitte (104, 105) aufweist, die mit dem Gewebe elektrisch in Kontakt bringbar sind und wobei der HF-Generator (18) einen ersten (15) und einen gegenpoligen zweiten Ausgang (14) aufweist, wobei
- in einem bipolaren Betrieb jeweils einer der Ausgänge (14, 15) mit jeweils einem Schlingenabschnitt (104, 105) verbunden wird und
- in einem monopolaren Betrieb der ersten Ausgang (15) mit einer am Patienten anbringbaren Neutralelektrode (17) und
- der zweiten Ausgang (14) mit mindestens einem Schlingenabschnitt (104, 105) verbunden wird,
**dadurch gekennzeichnet, dass**
weiterhin mindestens zeitweise ein monopolarer Betrieb eingestellt wird, bei welchem alternierend oder vom Operateur einstellbar alternativ jeweils einer der beiden Schlingenabschnitte (104 oder 105) mit dem zweiten Ausgang (14) verbunden ist.

2. Verfahren nach Anspruch 1,
**dadurch gekennzeichnet, dass**
der zweite Ausgang (14) selbsttätig alternierend mit einem der beiden Schlingenabschnitte (104 oder 105) verbunden wird.

3. Verfahren nach einem der vorhergehenden Ansprüche,
**dadurch gekennzeichnet, dass**
alternierend zwischen monopolarem und bipolarem Betrieb umgeschaltet wird.

4. Verfahren nach einem der vorhergehenden Ansprüche,
**dadurch gekennzeichnet,**
**dass** der HF-Generator (18) alternierend zwischen einer ersten Betriebsart, einem Schneidebetrieb, in welchem das Gewebe vornehmlich geschnitten wird, und einer zweiten Betriebsart, einem Koagulationsbetrieb, in welchem das Gewebe vornehmlich koaguliert wird, hin und her geschaltet wird.

5. Verfahren nach einem der vorhergehenden Ansprüche, insbesondere nach Anspruch 4,
**dadurch gekennzeichnet, dass**
im Koagulationsbetrieb der zweite Ausgang (14) mit beiden Schlingenabschnitten (104, 105) verbunden wird.

6. Verfahren nach einem der vorhergehenden Ansprüche insbesondere nach Anspruch 4,
**dadurch gekennzeichnet, dass**
im Koagulationsbetrieb die Schlinge (103) im bipolaren Betrieb betrieben wird.

7. HF-chirurgische Anordnung zum HF-chirurgischen Entfernen pathologischer Gewebe in Hohlorganen eines Patienten, z.B. von Polypen im Gastrointestinaltrakt, wobei die HF-chirurgische Anordnung ein HF-Chirurgiegerät (5) mit einem HF-Generator (18) zur Erzeugung des hierfür erforderlichen Hf-Stroms und eine bipolare Schlinge (103) umfasst, die zwei gegeneinander isolierte Schlingenabschnitte (104, 105) aufweist, die mit dem Gewebe elektrisch in Kontakt bringbar sind und wobei der HF-Generator (18) einen ersten (15) und einen gegenpoligen zweiten Ausgang (14) aufweist, wobei die Einrichtung Adapter (1, 20), Schalter (32) und/oder Wechselschalter (31, 42) oder dergleichen Umschalteinrichtung (30) umfasst, die
- in einem bipolaren Betrieb jeweils einen der Ausgänge (14, 15) mit jeweils einem Schlingenabschnitt (104, 105) verbindet und die
- in einem monopolaren Betrieb den ersten Ausgang (15) mit einer am Patienten anbringbaren Neutralelektrode (17) und
- den zweiten Ausgang (14) mit mindestens einem Schlingenabschnitt (104,105) verbindet,
**dadurch gekennzeichnet, dass**
die Anordnung weiterhin derart ausgebildet ist, dass mindestens zeitweise ein monopolarer Betrieb einstellbar ist, bei welchem alternierend oder vom Operateur einstellbar alternativ jeweils einer der beiden Schlingenabschnitte (104 oder 105) mit dem zweiten Ausgang (14) verbunden ist.

8. Anordnung nach Anspruch 7,
**dadurch gekennzeichnet, dass**
die Umschalteinrichtung (30) einen Adapter (1, 20) umfasst, der einen Steckkontakt (7) eines bipolaren Anschlusskabels (3) einer bipolaren Schlinge (103) mit einem ersten Kontaktelement (10) verbindet, der in eine monopolare Buchse (4) des HF-Chirurgiegerätes (5) einsteckbar ist.

9. Anordnung nach einem der Ansprüche 7 oder 8, insbesondere nach Anspruch 8,
**dadurch gekennzeichnet, dass**
die Umschalteinrichtung (30) einen Schalter (32) zum Verbinden der zwei Schlingenabschnitte (104, 105) miteinander und/oder einen ersten Wechselschalter (31) zum Verbinden mindestens eines Schlingenabschnittes (104, 105) mit dem zweiten Ausgang (14) umfasst.

10. Anordnung nach einem der Ansprüche 7-9
**dadurch gekennzeichnet, dass**
die Umschalteinrichtung (30) einen zweiten Wechselschalter (42) umfasst, zum alternierenden oder zum alternativen Verbinden des ersten Ausganges (15) mit der Neutralelektrode (17) oder mit mindestens einem Schlingenabschnitt (104, 105).

11. Anordnung nach einem der Ansprüche 7-10,
**dadurch gekennzeichnet, dass**
die Umschalteinrichtung (30) elektronisch gesteuert ist.

12. Anordnung nach einem der Ansprüche 7-11,
**dadurch gekennzeichnet, dass**
die Umschalteinrichtung (30) eine Steuereinrichtung (50) umfasst zum Steuern verschiedener Betriebsarten, z.B. Schneiden, Koagulieren, Softkoagulation des HF-Chirurgiegerätes (5).

## Claims

1. A method for operating an RF surgical arrangement for removing pathological tissue in hollow organs of patients, e.g. of polyps in the gastrointestinal tract, by RF surgery, wherein the RF surgical arrangement comprises an RF surgical device (5) having an RF generator (18) for generating the respective required RF current and a bipolar loop (103) which has two loop sections (104, 105) that are insulated from each other and that can be brought into electrical contact with the tissue, and wherein the RF generator (18) comprises a first (15) and a second output (14) of opposing polarity, wherein
- in bipolar operation one of the outputs (14, 15) is respectively connected to at least one loop section (104, 105), and
- in monopolar operation the first output (15) is connected to a neutral electrode (17) that can be attached to the patient, and
- the second output (14) is connected to at least one loop section (104, 105), **characterized in that**
monopolar operation is further set at least temporarily, in which only one of the two loop sections (104 or 105) is connected to the second output (14) in an alternating fashion or in an alternatively adjustable manner by the surgeon.

2. A method according to claim 1, **characterized in that** the second output (14) is connected in an automatically alternating fashion to one of the two loop sections (104 or 105).

3. A method according to one of the preceding claims, **characterized in that** switching is performed in an alternating fashion between monopolar and bipolar operation.

4. A method according to one of the preceding claims, **characterized in that** the RF generator (18) is switched in an alternating manner back and forth between a first operating mode, i.e. a cutting operation, in which the tissue is mainly cut, and a second operating mode, i.e. coagulation operation, in which the tissue is predominantly coagulated.

5. A method according to one of the preceding claims, especially according to claim 4, **characterized in that** in coagulation operation the second output (14) is connected to the two loop sections (104, 105).

6. A method according to one of the preceding claims, especially according to claim 4, **characterized in that** in coagulation operation the loop (103) is operated in bipolar operation.

7. An RF surgical arrangement for removing pathological tissue in hollow organs of patients, e.g. of polyps in the gastrointestinal tract, by RF surgery, wherein the RF surgical arrangement comprises an RF surgical device (5) having an RF generator (18) for generating the respective required RF current and a bipolar loop (103) which has two loop sections (104, 105) that are insulated from each other and that can be brought into electrical contact with the tissue, and wherein the RF generator (18) comprises a first (15) and a second output (14) of opposing polarity, wherein the device comprises an adapter (1, 20), switch (32) and/or changeover switch (31, 42) or a similar changeover device (30) which
- in bipolar operation respectively connects one of the outputs (14, 15) to at least one loop section (104, 105), and which
- in monopolar operation connects the first output (15) to a neutral electrode (17) that can be attached to the patient, and
- connects the second output (14) to at least one loop section (104, 105), **characterized in that**
the arrangement is further arranged in such a way that monopolar operation is adjustable at least temporarily, in which one of the two loop sections (104 or 105) is respectively connected to the second output (14) in an alternating fashion or in an alternatively adjustable manner by the surgeon.

8. An arrangement according to claim 7, **characterized in that** the changeover device (30) comprises an adapter (1, 20) which connects a plug contact (7) of a bipolar connecting cable (3) of a bipolar loop (103) to a first contact element (10) which can be inserted into a monopolar socket (4) of the RF surgical device (5).

9. An arrangement according to one of the claims 7 or 8, especially according to claim 8, **characterized in that** the changeover device (30) comprises a switch (32) for connecting the two loop sections (104, 105) to each other and/or a first changeover switch (31) for connecting at least one loop section (104, 105) to the second output (14).

10. An arrangement according to one of the claims 7 to 9, **characterized in that** the changeover device (30) comprises a second changeover switch (42) for alternating or alternative connection of the first output (15) to the neutral electrode (17) or the at least one loop section (104, 105).

11. An arrangement according to one of the claims 7 to 10, **characterized in that** the changeover device (30) is electronically controlled.

12. An arrangement according to one of the claims 7 to 11, **characterized in that** the changeover device (30) comprises a control device (50) for controlling various operating modes, e.g. cutting, coagulating, soft coagulation of the RF surgical device (5).

## Revendications

1. Procédé de pilotage d'un dispositif de chirurgie à haute fréquence pour l'ablation par chirurgie à haute fréquence de tissus pathologiques dans les organes creux d'un patient, par exemple de polypes dans le tractus gastro-intestinal, le dispositif de chirurgie à haute fréquence comprenant un appareil de chirurgie à haute fréquence (5) avec un générateur haute fréquence (18) pour la génération du courant à haute fréquence nécessaire à cette fin et une boucle bipolaire (103) comportant deux parties de boucle (104, 105) isolées l'une de l'autre, qui peuvent être mises en contact électrique avec le tissu, et le générateur haute fréquence (18) comportant une première sortie (15) et une deuxième sortie (14) de polarité opposée, dans lequel
- une des sorties (14, 15) à la fois est connectée à une partie de boucle (104, 105) à la fois dans un mode bipolaire et
- la première sortie (15) avec une électrode neutre (17) pouvant être appliquée sur le patient dans un mode unipolaire et
- la deuxième sortie (14) est connectée à au moins une partie de boucle (104, 105), **caractérisé en ce qu'**est au moins temporairement établi un mode unipolaire dans lequel une des deux parties de boucle (104 ou 105) à la fois est connectée alternativement à la deuxième sortie (14) de façon alternée ou réglable par le chirurgien.

2. Procédé selon la revendication 1, **caractérisé en ce que** la deuxième sortie (14) est connectée automatiquement en alternance avec une des deux parties de boucle (104 ou 105).

3. Procédé selon l'une des revendications précédentes, **caractérisé en ce que** les modes unipolaire et bipolaire commutent alternativement.

4. Procédé selon l'une des revendications précédentes, **caractérisé en ce que** le générateur haute fréquence (18) alterne en va-et-vient entre un premier mode de fonctionnement, un mode de coupe, dans lequel le tissu est principalement coupé, et un deuxième mode de fonctionnement, un mode de coagulation, dans lequel le tissu est principalement coagulé.

5. Procédé selon l'une des revendications précédentes, en particulier selon la revendication 4, **caractérisé en ce qu'**en mode de coagulation, la deuxième sortie (14) est connectée aux deux parties de boucle (104, 105).

6. Procédé selon l'une des revendications précédentes, en particulier selon la revendication 4, **caractérisé en ce qu'**en mode de coagulation, la boucle (103) fonctionne en mode bipolaire.

7. Dispositif de chirurgie à haute fréquence pour l'ablation par chirurgie à haute fréquence de tissus pathologiques dans les organes creux d'un patient, par exemple de polypes dans le tractus gastro-intestinal, lequel dispositif de chirurgie à haute fréquence comprend un appareil de chirurgie à haute fréquence (5) avec un générateur haute fréquence (18) pour la génération du courant à haute fréquence nécessaire à cette fin et une boucle bipolaire (103) comportant deux parties de boucle (104, 105) isolées l'une de l'autre, qui peuvent être mises en contact électrique avec le tissu, le générateur haute fréquence (18) comportant une première sortie (15) et une deuxième sortie (14) de polarité opposée,
dans lequel le dispositif comprend des adaptateurs (1, 20), des commutateurs (32) et/ou des commutateurs va-et-vient (31, 42) ou un dispositif de commutation (30) similaire qui
- connecte une des sorties (14, 15) à la fois à une partie de boucle (104, 105) à la fois dans un mode bipolaire et
- la première sortie (15) avec une électrode neutre (17) pouvant être appliquée sur le patient dans un mode unipolaire et
- connecte la deuxième sortie (14) à au moins une partie de boucle (104, 105),
**caractérisé en ce que** le dispositif est conçu de telle façon que puisse être établi au moins temporairement un mode unipolaire dans lequel une des deux parties de boucle (104 ou 105) à la fois est connectée alternativement à la deuxième sortie (14) de façon alternée ou réglable par le chirurgien.

8. Dispositif selon la revendication 7, **caractérisé en ce que** le dispositif de commutation (30) comprend un adaptateur (1, 20) qui connecte un contact enfichable (7) d'un câble de raccordement bipolaire (3) d'une boucle bipolaire (103) à un premier élément de contact (10) qui peut être enfiché dans une prise unipolaire (4) de l'appareil de chirurgie à haute fréquence (5).

9. Dispositif selon la revendication selon l'une des revendications 7 ou 8, en particulier selon la revendication 8, **caractérisé en ce que** le dispositif de commutation (30) comprend un commutateur (32) destiné à connecter les deux parties de boucle (104, 105) l'une à l'autre et/ou un premier commutateur va-et-vient (31) destiné à connecter au moins une partie de boucle (104, 105) à la deuxième sortie (14).

10. Dispositif selon l'une des revendications 7 à 9, **caractérisé en ce que** le dispositif de commutation (30) comprend un deuxième commutateur va-et-vient (42) pour la connexion alternée ou alternative de la première sortie (15) à l'électrode neutre (17) ou à au moins une partie de boucle (104, 105).

11. Dispositif selon l'une des revendications 7 à 10, **caractérisé en ce que** le dispositif de commutation (30) est à commande électronique.

12. Dispositif selon l'une des revendications 7 à 11, **caractérisé en ce que** le dispositif de commutation (30) comprend un dispositif de commande (50) pour commander différents modes de fonctionnement de l'appareil de chirurgie à haute fréquence (5), par exemple coupe, coagulation, coagulation douce.
